# EUROPEAN PATENT SPECIFICATION

(11) **EP 4 215 242 B1**
(45) Date of publication and mention of the grant of the patent: **12.11.2025**
(21) Application number: 22152263.4
(22) Date of filing: 19.01.2022
(51) Int. Cl.: A61N 5/10

(54) **A METHOD AND A COMPUTER PROGRAM PRODUCT AND A COMPUTER SYSTEM FOR USE WITH A RADIATION THERAPY DELIVERY SYSTEM**
VERFAHREN UND COMPUTERPROGRAMMPRODUKT UND COMPUTERSYSTEM ZUR VERWENDUNG MIT EINEM STRAHLENTHERAPIEABGABESYSTEM
PROCÉDÉ ET PRODUIT PROGRAMME INFORMATIQUE ET SYSTÈME INFORMATIQUE DESTINÉ À ÊTRE UTILISÉ AVEC UN SYSTÈME D'ADMINISTRATION DE RADIOTHÉRAPIE

(43) Date of publication of application: 26.07.2023
(73) Proprietor: RaySearch Laboratories AB, 104 30 Stockholm (SE)
(72) Inventor: TRANEUS, Erik, 103 65 STOCKHOLM (SE); HÅRDEMARK, Björn, 103 65 STOCKHOLM (SE)

(56) References cited:
- US-A1- 2015 124 930
- US-A1- 2019 054 320

## Description

### Technical Field

The present invention relates to radiation therapy delivery systems for scanned proton beams, and in particular to quality assurance of treatment delivered from such systems.

### Background

A goal of radiation therapy, or radiotherapy is typically to deliver sufficient dose to a target such as a tumor, to eliminate, or at least reduce the size of the tumor. Radiation therapy delivery systems for delivering scanned ion beams to patients are known in the art. The ions are typically protons, but other types of ions may be used as well. In this context, reference is made to document US2019/054320 A1.

Radiotherapy treatment always involves delivering dose outside of the target, to healthy tissue or organs at risk, and therefore there is always a risk that healthy organs or tissue are damaged by the radiation. One emerging treatment method that appears to involve less undesired damage is FLASH therapy, which involves treatment at a much higher dose rate than conventional therapy, for example, 70 Gy/s. In the literature related to FLASH, various lower limits to the dose rates are suggested, such as at least 40 Gy/s or 50 Gy/s. If, for example, a dose of 20 Gy is to be delivered with a dose rate of 70 Gy/s, the whole dose will be delivered in 0.29 s. In contrast, conventional radiotherapy treatment is delivered at much lower dose rates, a typical dose rate for conventional radiotherapy treatment being a few Gy per minute.

It has been found that with FLASH therapy the damage done to healthy tissue by a particular dose is lower than with conventional therapy, while the effect on the target, that is, the tumorous tissue response, remains the same. Therefore, FLASH therapy can have significant advantage over conventional radiotherapy treatment, since they can affect the tumor with less damage done to the healthy tissue.

The FLASH effect is highly dependent on the time structure of the radiation delivery, to ensure that the dose rate is high enough to activate the FLASH effect.

Therefore, to ensure that a treatment plan has the intended effect on the patient, it is vital to know the time structure of the beam delivery. The rate of energy deposition in a voxel depends on a number of factors, including
- the scanning characteristics of the delivery system
- the beam current
- the beam quality
- the treatment plan

When delivering FLASH treatments by scanned proton beams the dose is the resulting accumulation of energy depositions. The energy deposition over time is non-trivial and depends on the rate of energy deposition in a voxel, which is a function of the scanning characteristics of the delivery system, the beam current, the treatment plan and the beam quality.

Normally, a prediction of the system characteristics may be provided by the system vendors, in the form of nominal data or a model of the system. This may not always be the case, and even if it is, there may still be a desire to verify the data to ensure that treatment delivered to a patient will have the intended properties. As explained above, this is particularly important in the case of FLASH therapy, where the time structure is essential for achieving the desired effect.

### Summary of the invention

The invention is defined in the appended set of claims. Methods mentioned hereinafter and related to therapeutic treatment of a living human body do not form part of the present invention. The invention relates to a computer-based method for use in a radiotherapy treatment delivery system arranged to deliver ion radiotherapy treatment according to a plan, without a patient present, comprising the steps of
- obtaining a time-resolved simulation of the quantity representative of the beam delivery sequence for delivery of the plan according to a model of the system,
- obtaining a time-resolved measurement of a quantity representative of a beam delivery sequence for delivery of the plan related to the delivery of the plan,
- comparing data related to the time-resolved measurement to the time-resolved simulation,
- taking action in dependence of the result of the comparison, wherein the action includes at least one of determining whether to adapt the model, or the plan, or diagnosing the function of the delivery system.

The plan may be a treatment plan arranged for delivery to a patient, or a test plan, which is normally simpler than a treatment plan and used to test aspects of the delivery system. Adapting the model may involve changing the model itself or adjusting one or more parameters of the model.

The step of comparing includes comparing the time-resolved measurement to the time-resolved simulation.

The method further comprises the steps of
- obtaining simulated dose information based on the time-resolved simulation,
- obtaining measured dose information based on the time-resolved measurement, wherein the step of comparing includes comparing the simulated dose information to the measured dose information, the dose information including information regarding at least one of physical dose, RBE weighted dose, and FLASH dose resulting from the plan.

The Relative Biological Effectiveness (RBE) weighted dose means the effective dose to the patient, which may differ from the physical dose, influenced by the dose rate, type of radiation and other factors as is known to the skilled person. The FLASH dose is a special case of RBE weighted dose, in which very high dose rates causes even greater changes between the physical dose and the RBE weighted dose.

In some preferred embodiments, the time-resolved measurement is obtained from a dry-run of the plan. This enables the adjustment of model parameters before actual delivery of a treatment plan to the patient.

In other embodiments, the time-resolved measurement is obtained during delivery of the plan to a patient. In this case, data obtained from the measurement may be used to adjust the remaining fractions of the plan based on any deviations between the dose according to the plan and the dose actually delivered.

As is common in the art, the treatment plan may be designed to be delivered to the patient in a number of fractions, wherein the time-resolved simulation is obtained for one or more of these fractions. In these cases, simulations may be obtained at different stages of the treatment, typically shortly before the delivery of one of the fractions, to account for the fact that changes in environmental factors such as temperature and atmospheric pressure may affect the simulation.

The time-resolved measurement may be obtained during delivery of the same one or more of the fractions as the time-resolved simulation, wherein the step of taking action includes adapting delivery of a subsequent fraction. This is particularly useful if the measurement is obtained as a dry run of the system delivering the subsequent fraction. In this case, the dry run may be used to obtain information about the current function of the system and what will be the result of delivery of the subsequent fraction, which may be used to adapt the subsequent fraction to the current function of the system.

Preferably, the time-resolved measurement is obtained during delivery of the same one or more of the fractions as the time-resolved simulation.

The method may include the steps of obtaining delivered FLASH dose information from the time-resolved measurement and simulated FLASH dose information from the time-resolved simulation and the step of comparing includes comparing the delivered FLASH dose information to the simulated FLASH dose information, and the step of taking action includes amending the plan for subsequent fractions.

The step of comparing may include comparing accumulated dose information from one fraction or from a number of fractions. If the time-resolved measurement is obtained from a dry-run before the delivery of a particular fraction, the result of the comparison may be used to adapt that particular fraction in view of the difference.

The step of taking action may include adjusting the model based on the result of the comparison.

The step of taking action may include assessing the function of the delivery system based on the result of the comparison.

The measuring may be performed in any suitable manner and using any suitable detector. For example, a detector such as a radiation detector array or a laterally integrating detector may be used.

The model of the system may include parameter values that are based on nominal data provided for the delivery system. Such parameter values are normally provided by the vendor of the system. Alternatively, the model includes data obtained by an experimental characteristic of the system. This means that during parameter values are determined based on testing of the delivery system. In both cases, the method enables verification and/or adjustment of the parameters of the model. In both cases, the method may also be used to adjust the model itself.

The disclosure also relates to a computer program product comprising computer readable code means which, when run in a computer, will cause the computer to perform the method according to any one of the embodiments discussed in this document. The computer program product may include non-transitory storage medium on which the code means is stored.

The disclosure also relates to a computer system comprising a processor, a program memory and optionally a data memory, wherein the program memory comprises such a computer program product.

### Brief description of drawings

The invention will be described in more detail in the following, by way of examples and with reference to the appended drawings.
Figures 1a and 1b illustrate a dose rate and dose accumulation for a voxel over a time period of 500 ms.
Fig. 2 is a general flow chart of a method according to an embodiment of the invention
Fig. 3 is a flow chart of a method according to a second embodiment.
Fig. 4 is a flow chart of a method according to a third embodiment.
Fig. 5 is a flow chart of a method according to a third embodiment.
Fig. 6 shows a computer system in which the disclosed method can be implemented.

### Detailed description of embodiments

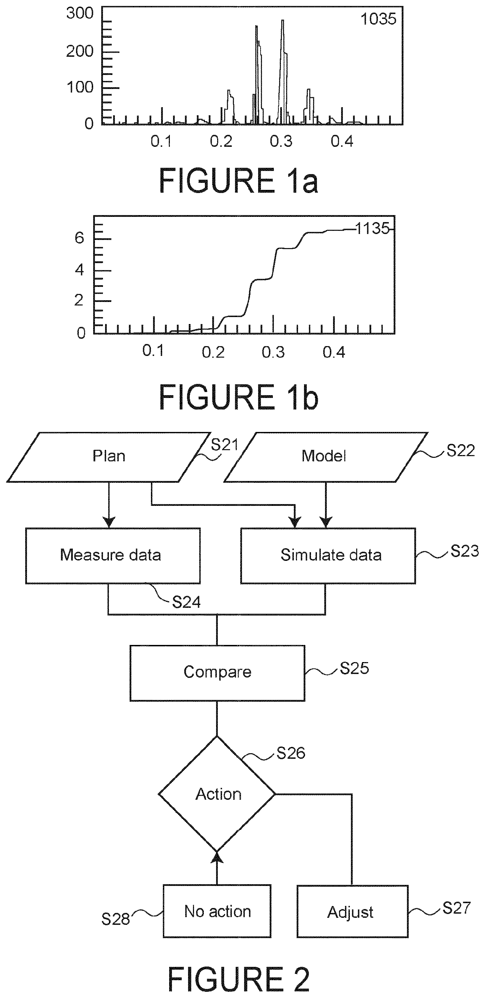

A typical dose rate and dose accumulation time trace of a voxel in a PBS beam is show in Figures 1a and 1b. As shown in Figure 1a, the voxel receives several dose contributions during the 500 ms period, the dose rate varying because spots directed to a location near the voxel will affect the voxel differently depending on how close they are. As can be seen, the dose rate of the four highest peaks varies from about 100 Gy/s to about 300 Gy/s, but each peak is only a few milliseconds long. The peaks are only a few milliseconds apart, which means that they will cooperate to produce an enhanced FLASH effect. Figure 1b is a corresponding diagram showing the accumulated dose for the same voxel. The modelling of the FLASH tissue sparing effect may require such time traces as input data. Thus, it is essential to be able to predict and measure such data.

According to the present disclosure, it is desired to obtain such time trace data, related to dose rate or dose accumulation, or both, to be used as input data when modelling the effect of the treatment plan, in particular of a FLASH treatment plan. Such time trace data may be acquired in a number of different ways. For example, in a proton beam scanning delivery system, the plan includes a number of energy levels and a number of spots within each energy level, as the skilled person is aware.

Figure 2 is a flow chart of a general embodiment of a method according to the invention. Input data to the method include an ion-based radiotherapy treatment plan S21, which has been obtained in any suitable manner and a model S22 of a treatment delivery system to be used to deliver the plan. The model may be provided by a vendor of the system, or obtained in another way. In step S23, a simulated signal related to delivery of the treatment plan is obtained based on the plan S21 and the model S22. In step S24, a measured signal of related to the delivery of the treatment plan using the delivery system is obtained by measurement of a beam delivery sequence. The beam delivery sequence may involve delivery of the plan to a patient, or may be a dry run without a patient present. The simulated signal and the measured signal are both time-resolved signals reflecting a quantity representative of the beam delivery sequence for delivery of the treatment plan S21, based on the model and the measurements, respectively. Instead of a treatment plan S21, the plan may be a test plan designed to test the function of the system but not suitable for delivery to a patient.

In step S25, the simulated signal and the measured signal are compared. Step S26 is a decision step for deciding which action to take in dependence of the result of the comparison. The outcome may be that some action needs to be taken S27. As will be discussed in more detail below, the actions may include adjusting the model parameters or improving the model itself, adapting the plan, or determining that the delivery system needs to be adjusted. The result of the comparison may also be that no action needs to be taken S28.

In each of the embodiments in this document, the model S22, S32, S42, S52 of the treatment delivery system may be a system model including nominal parameters provided by the vendor of the delivery system. It may also be based on an experimental characterization of the system. The model includes parameter values that likewise may be provided by the vendor of the delivery system or based on an experimental characterization of the system. The simulated signal in S23, S33, S43, S53 therefore represents a simulation of the treatment delivery system, which may deviate from the actual behaviour of the treatment delivery system.

The measuring may be performed using any suitable form of detector, typically a type of ion chamber detector. Such detectors are available as detector arrays and as laterally integrating detectors arranged to measure a total signal across the beam. The measured quantity may include the beam current, and/or one or more other parameters that change with time may be used in the delivery data, for example, one or more of the following:
- the current to the magnets controlling the beams. This will be particularly useful for comparing measured data with modelled or nominal data.
- gantry angle, in the case of ARC therapy.

In each of the embodiments in this document, the time resolved measurement S24, S34, S44, S54 may be performed as a dry-run of the plan using the treatment delivery system. A dry-run is performed without a patient present, and may be performed with or without a phantom. Another way of obtaining such data is during delivery of a plan to a patient. In this case a measuring device must be provided near the patient to measure the actual radiation provided, for example beam current as a function of time. The measurements must have a sufficiently high time resolution to enable the desired radiobiological modeling, preferably at least to one millisecond. Even more preferred would be a fraction of a millisecond, for example 1/10 of a millisecond. In both cases, the time-resolved measurement represents the actual beam-delivery sequence from the treatment delivery system.

In both the above cases, the measurement can be made by methods that are known per se, for example, by picking up the signal from one of the existing ion chamber monitors or by inserting a dedicated and independent detector in the beam or in a phantom being irradiated. The measurements may be made over a suitable time period matching the time of radiation delivery. The detector may typically comprise an array of detectors, each arranged to detect the time when it receives dose, and the magnitude of the dose received. Alternatively, a single ion chamber detector may be used, such as a broad plane parallel ion chamber that measures the current across the whole beam. Both radiation detector arrays and single ion chamber detectors are available to the skilled person. By matching the plan data (spot data and energy layer data) with the time trace an accurate plan delivery timing characterization will be possible. The data will have essentially the same structure as shown in Figs. 1a and 1b.

As is common in the art, the treatment plan may be divided into a number of treatment fractions, to be delivered to the patient at certain time intervals, for example at the rate of one fraction per day. The simulation in step S23, S33, S43, S53 and the measurement in step S24, S34, S44, S54 may be performed for a complete plan including one or more fractions. Alternatively, the simulation and measuremen steps may be performed for one or more fractions.

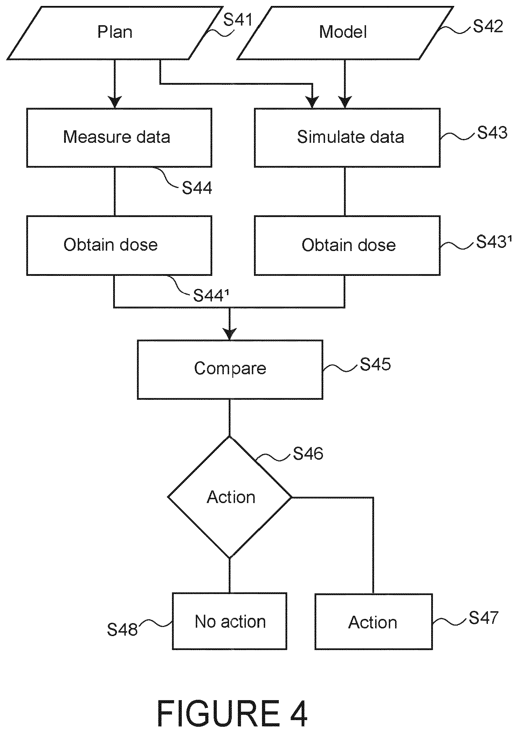

As will be discussed below, the comparison may be performed by comparing the simulated time-resolved signal to the time-resolved measured signal, or the signals may be processed in a suitable way before comparison. For example, as will be discussed in more detail in connection with Figure 4, the delivered doses according to the simulated time-resolved signal and according to the measured time-resolved signal may be calculated and the doses may be compared. The doses may be calculated a physical doses. In many cases it will be more relevant to calculate the doses as RBE doses, taking into account the effective dose in each particular case. As explained above, the RBE dose may be different from the physical dose depending on factors such as radiation type, dose rate, total dose, and other factors. If the treatment involves FLASH treatment, the doses may be calculated as FLASH doses, to reflect the FLASH effect resulting from the beam delivery sequence. This is a special case of RBE, since the very high dose rates used in FLASH therapy causes large deviations between the physical dose and the RBE dose. As mentioned above, the FLASH effect is highly dependent on the time structure of the treatment delivery, so differences between the model and the actual system properties may cause significant deviations between the dose according to the plan and the actual dose to the patient.

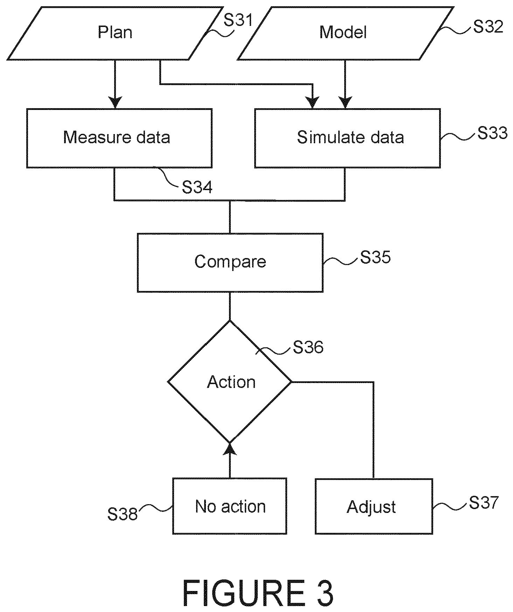

Figure 3 illustrates a specific embodiment of the method according to the invention, which may be used to evaluate the function of the treatment delivery system. This is suitably done at regular time intervals, for example once a week, to ensure that the system is functioning as it should. As in Figure 2, input data include a treatment plan S31 and a system model S32. In this case, the treatment plan, can be a simple plan, not intended for delivery, for example, just a uniform beam. As in Figure 2, a simulated time-resolved signal is obtained based on the treatment plan S31 and the system model S32. A measured time-resolved signal is obtained S34 during delivery of the plan. Step S34 will typically be performed during a dry run. In step S35, the simulated time resolved signal and the measured time-resolved signal are compared. Step S36 is a decision step. If the comparison in step S35 shows a system malfunction, action is taken in Step S37, for example an operator is alerted that the system needs adjustment. If the comparison shows that the measured time-resolved signal and the simulated time-resolved signal match, no action needs to be taken, step S38.

Figure 4 illustrates another embodiment of the method according to the invention. As before, input data include a treatment plan S41 and a system model S42. As in Figures 2 and 3, a simulated time-resolved signal is obtained in step S43 based on the treatment plan S41 and the system model S42 and a measured time-resolved signal is obtained in step S44. In this embodiment the simulated signal is processed in step S43' to obtain simulated dose information about the dose resulting from the plan according to the system model S42. Similarly, the measured signal is processed in step S44' to obtain measured dose information about the dose resulting from the plan according to the measured data. In step S45, the simulated dose information and the measured dose information are compared. Step S45 may optionally also include comparing the simulated and measured signals as in step S25. If the comparison shows that the measured dose information deviates from the simulated dose information by more than a certain threshold, decision step S46 determines that action needs to be taken in step S47. Or if the measured and simulated dose information match, no action may be taken in step S48. In this embodiment the action taken in step S47 may involve one or more of the following:
- a signal may be issued to alert the operator that the actual delivered dose that will be the result of delivery of the treatment plan will deviate from the planned dose because of a deviation in the system model
- a comparison of the simulated signal and the measured signal obtained in steps S43 and S44 may be performed, to help identify the source of the deviation

For each of the embodiments discussed in connection with Figures 2, 3 and 4, the treatment plan may be designed to be delivered all at once or in one or more fractions. In the latter case, the simulation in step 23, 33, 43 and the measurement in steps 23, 33, 43 may relate to all fractions or just one, or a number of fractions.

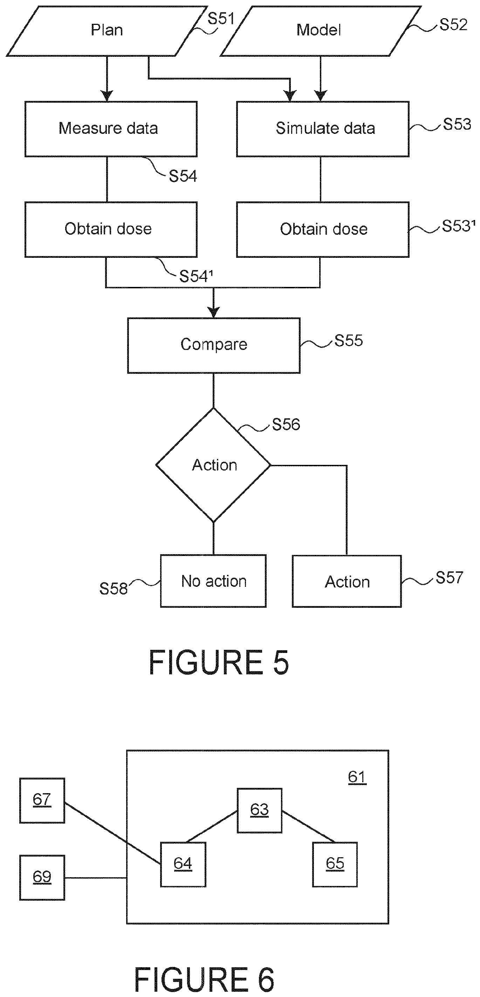

Figure 5 illustrates a third embodiment, to enable dose tracking and plan adaptation when the treatment plan is designed to be delivered in a number of fractions. In such cases it is relevant to evaluate the dose and the adjusted dose delivered to the patient during one fraction, and accumulated for all fractions that have been delivered, and to use this data to adapt the plan for the remaining fractions.

As before, the input data include a treatment plan S51 and a model S52 of the treatment delivery system. A simulated signal that is a time- resolved measured signal of a quantity representative of a beam delivery sequence for delivery of one or more of the fractions of the plan is obtained in step S53. A measured signal that is a time- resolved measured signal of a quantity representative of a beam delivery sequence for delivery of the one or more of the fractions of the plan is obtained in step S54. Optionally, steps S53' and S54' may be included to calculate simulated dose information and measured dose information based on the simulated signal and the measured signal, respectively, in the same way as in steps S43' and S44'. In step S55, the simulated signal and the measured signal, and/or the simulated and measured doses, are compared. Step S56 is a decision step in which the need for further action is determined.

In a first implementation of the third embodiment, the measured signal is obtained in step S54 during a dry run of a considered fraction that has not yet been delivered to the patient, preferably a fraction that is to be delivered shortly after the measurement, for example on the same day. In this case, the result of the comparison in step S55 will be an indicator of how the system will perform the delivery and what will be the actual dose resulting from the considered fraction. The dry run may also consider more than one fraction. In this implementation, the result of the comparison can be used to change the considered fraction, and possibly other remaining fractions, to adjust to the system's current properties. It can also be used to determine that a whole new plan needs to be obtained, if the simulated signal and the measured signal are too different.

In a second implementation of the third embodiment, the measured signal is obtained in step S54 may have been obtained during delivery of one or more previous fractions, or as a dry run of the one or more previous fraction or fractions. In step S54', therefore, actual accumulated dose information from the previous fraction or fractions is obtained, based either on a dry run of the system or on actual delivery of the fractions to the patient. The simulated accumulated dose is calculated in step S53' based on the simulated signal. The information regarding the actual accumulated dose may be used to recalculate the remaining fractions, to take into account the difference between the actual accumulated dose and the simulated accumulated dose.

Fig. 6 is a schematic overview of a computer system in which the method according to the disclosure may be carried out. A computer 61 comprises a processor 63, a data memory 64 and a program memory 65. Preferably, one or more user input/output means 67, 68 is also present, in the form of a keyboard, a mouse, a joystick, voice recognition means and/or any other available user input means, and a display, speaker etc. for output. The user input means may also be arranged to receive data from an external memory unit.

The data memory 64 holds data that may be used in the method, such as the nominal system data provided by the vendor, one or more treatment plans, and/or simulation results. As will be understood, the data memory 64 is only shown schematically. There may be several data memory units, each holding one or more different types of data.

The program memory 65 holds a computer program arranged to control the processor to perform the procedure according to any of the embodiments discussed above. Like the data memory 64, the program memory may also be implemented as one or several units as is seen fit.

## Claims

1. A computer-based method for testing a radiotherapy treatment delivery system arranged to deliver ion radiotherapy treatment as scanned ion beams according to a plan, the method comprising the steps, without a patient present, of
• obtaining a time-resolved simulation of a quantity representative of the beam delivery sequence for delivery of the plan according to a model of the system,
• obtaining a time-resolved measurement of the quantity representative of a beam delivery sequence for delivery of the plan related to the delivery of the treatment plan,
• comparing data related to the time-resolved measurement to the time-resolved simulation,
• taking action in dependence of the result of the comparison, wherein the action includes at least one of determining whether to adapt the model, or the plan, or diagnosing the function of the delivery system.

2. A method according to claim 1, wherein the step of comparing includes comparing the time-resolved measurement to the time-resolved simulation.

3. A method according to claim 1 or 2, further comprising the steps of
• obtaining simulated dose information based on the time-resolved simulation,
• obtaining measured dose information based on the time-resolved measurement, wherein the step of comparing includes comparing the simulated dose information to the measured dose information, the dose information including information regarding at least one of physical dose, RBE weighted dose, and FLASH dose resulting from the plan.

4. A method according to any one of the preceding claims, wherein the time-resolved measurement is obtained based on a dry-run of the plan.

5. A method according to any one of the claims 1 - 3, wherein the plan is a treatment plan and the time-resolved measurement is obtained based on delivery of the treatment plan to a phantom.

6. A method according to claim 5, wherein the treatment plan is designed to be delivered to the phantom in two or more fractions, wherein the time-resolved simulation is obtained for one or more of these fractions.

7. A method according to claim 6, wherein the time-resolved measurement is obtained during delivery of the same one or more of the fractions as the time-resolved simulation, wherein the step of taking action includes adapting delivery of one or more subsequent fractions.

8. A method according to claim 7, comprising the steps of obtaining delivered FLASH dose information from the time-resolved measurement and simulated FLASH dose information from the time-resolved simulation and the step of comparing includes comparing the delivered FLASH dose information to the simulated FLASH dose information.

9. A method according to any one of the claims 6 - 8, wherein the step of comparing includes comparing accumulated dose information from a number of fractions.

10. A method according to any one of the preceding claims, wherein the step of taking action includes adjusting the model of the system based on the result of the comparison.

11. A method according to claim 3, wherein the step of taking action includes assessing the function of the delivery system based on the result of the comparison.

12. A method according to claim 1, wherein the model of the system includes parameters based on nominal data provided for the delivery system or the model of the system includes parameters obtained by an experimental characterization of the system.

13. A computer program product comprising computer readable code means which, when run in a computer, will cause the computer to perform the method according to any one of the preceding claims.

14. A computer system comprising a processor, a program memory and optionally a data memory, wherein the program memory comprises a computer program product according to claim 13.

## Patentansprüche

1. Computergestütztes Verfahren zum Testen eines Strahlentherapiebehandlungsabgabesystems, das eingerichtet ist, um eine Ionenstrahlentherapiebehandlung als gescannte Ionenstrahlen gemäß einem Plan abzugeben, das Verfahren umfassend die Schritte, ohne dass ein Patient anwesend ist,
• Erhalten einer zeitaufgelösten Simulation einer Größe, die für die Strahlabgabesequenz für eine Abgabe des Plans gemäß einem Modell des Systems repräsentativ ist,
• Erhalten einer zeitaufgelösten Messung der Größe, die für eine Strahlabgabesequenz für die Abgabe des Plans bezüglich der Abgabe des Behandlungsplans repräsentativ ist,
• Vergleichen von Daten bezüglich der zeitaufgelösten Messung mit der zeitaufgelösten Simulation,
• Ergreifen von Maßnahmen in Abhängigkeit von dem Ergebnis des Vergleichs, wobei die Maßnahmen mindestens eines von einem Bestimmen, ob das Modell oder der Plan angepasst werden soll, oder einem Diagnostizieren der Funktion des Abgabesystems einschließen.

2. Verfahren nach Anspruch 1, wobei der Schritt des Vergleichens das Vergleichen der zeitaufgelösten Messung mit der zeitaufgelösten Simulation einschließt.

3. Verfahren nach Anspruch 1 oder 2, ferner umfassend die Schritte
• Erhalten simulierter Dosisinformationen basierend auf der zeitaufgelösten Simulation,
• Erhalten von gemessenen Dosisinformationen basierend auf der zeitaufgelösten Messung, wobei der Schritt des Vergleichens das Vergleichen der simulierten Dosisinformationen mit den gemessenen Dosisinformationen einschließt, wobei die Dosisinformationen Informationen hinsichtlich mindestens einer von einer physikalischen Dosis, einer RBE-gewichteten Dosis und einer FLASH-Dosis, die aus dem Plan resultieren, einschließen.

4. Verfahren nach einem der vorstehenden Ansprüche, wobei die zeitaufgelöste Messung basierend auf einem Probelauf des Plans erhalten wird.

5. Verfahren nach einem der Ansprüche 1 bis 3, wobei der Plan ein Behandlungsplan ist und die zeitaufgelöste Messung basierend auf der Abgabe des Behandlungsplans an ein Phantom erhalten wird.

6. Verfahren nach Anspruch 5, wobei der Behandlungsplan konzipiert ist, um an das Phantom in zwei oder mehreren Fraktionen abgegeben zu werden, wobei die zeitaufgelöste Simulation für eine oder mehrere dieser Fraktionen erhalten wird.

7. Verfahren nach Anspruch 6, wobei die zeitaufgelöste Messung während der Abgabe der gleichen einen oder mehreren der Fraktionen wie die zeitaufgelöste Simulation erhalten wird, wobei der Schritt des Ergreifens von Maßnahmen ein Anpassen der Abgabe einer oder mehrerer nachfolgender Fraktionen einschließt.

8. Verfahren nach Anspruch 7, umfassend die Schritte des Erhaltens von Informationen einer abgegebenen FLASH-Dosis aus der zeitaufgelösten Messung und von Informationen einer simulierten FLASH-Dosis aus der zeitaufgelösten Simulation und wobei der Schritt des Vergleichens das Vergleichen der Informationen der abgegebenen FLASH-Dosis mit den Informationen der simulierten FLASH-Dosis einschließt.

9. Verfahren nach einem der Ansprüche 6 bis 8, wobei der Schritt des Vergleichens das Vergleichen der Informationen einer akkumulierten Dosis aus einer Anzahl von Fraktionen einschließt.

10. Verfahren nach einem der vorhergehenden Ansprüche, wobei der Schritt des Ergreifens von Maßnahmen das Anpassen des Modells des Systems basierend auf dem Ergebnis des Vergleichs umfasst.

11. Verfahren nach Anspruch 3, wobei der Schritt des Ergreifens von Maßnahmen ein Beurteilen der Funktion des Abgabesystems basierend auf dem Ergebnis des Vergleichs einschließt.

12. Verfahren nach Anspruch 1, wobei das Modell des Systems Parameter einschließt, die auf Nenndaten basieren, die für das Abgabesystem bereitgestellt werden, oder das Modell des Systems Parameter einschließt, die durch eine experimentelle Charakterisierung des Systems gewonnen werden.

13. Computerprogrammprodukt, umfassend computerlesbare Codemittel, die, wenn sie auf einem Computer ablaufen, den Computer veranlassen werden, das Verfahren nach einem der vorstehenden Ansprüche durchzuführen.

14. Computersystem, umfassend einen Prozessor, einen Programmspeicher und optional einen Datenspeicher, wobei der Programmspeicher ein Computerprogrammprodukt nach Anspruch 13 umfasst.

## Revendications

1. Procédé assisté par ordinateur permettant de tester un système de délivrance de traitement de radiothérapie agencé pour délivrer un traitement de radiothérapie ionique en tant que faisceaux ioniques balayés selon un plan, le procédé comprenant les étapes, sans patient présent, consistant à
• obtenir une simulation à résolution temporelle d'une quantité représentative de la séquence de délivrance de faisceau pour la délivrance du plan selon un modèle du système,
• obtenir une mesure à résolution temporelle de la quantité représentative d'une séquence de délivrance de faisceau pour la délivrance du plan, apparentée à la délivrance du plan de traitement,
• comparer des données apparentées à la mesure à résolution temporelle à la simulation à résolution temporelle,
• effectuer une action en dépendance du résultat de la comparaison, dans lequel l'action comporte au moins l'un parmi le fait de déterminer s'il faut adapter le modèle, ou le plan, ou le fait de diagnostiquer la fonction du système de délivrance.

2. Procédé selon la revendication 1, dans lequel l'étape de comparaison comporte la comparaison de la mesure à résolution temporelle à la simulation à résolution temporelle.

3. Procédé selon la revendication 1 ou 2, comprenant en outre les étapes consistant à
• obtenir des informations de dose simulée en fonction de la simulation à résolution temporelle,
• obtenir des informations de dose mesurée en fonction de la mesure à résolution temporelle, dans lequel l'étape de comparaison comporte la comparaison des informations de dose simulée aux informations de dose mesurée, les informations de dose comportant des informations concernant au moins l'une parmi une dose physique, une dose à pondération RBE, et une dose FLASH résultant du plan.

4. Procédé selon l'une quelconque des revendications précédentes, dans lequel la mesure à résolution temporelle est obtenue en fonction d'un essai à blanc du plan.

5. Procédé selon l'une quelconque des revendications 1 à 3, dans lequel le plan est un plan de traitement et la mesure à résolution temporelle est obtenue en fonction de la délivrance du plan de traitement à un fantôme.

6. Procédé selon la revendication 5, dans lequel le plan de traitement est conçu pour être délivré au fantôme en deux fractions ou plus, dans lequel la simulation à résolution temporelle est obtenue pour une ou plusieurs de ces fractions.

7. Procédé selon la revendication 6, dans lequel la mesure à résolution temporelle est obtenue pendant la délivrance de la même ou des mêmes fractions que la simulation à résolution temporelle, dans lequel l'étape consistant à effectuer une action comporte l'adaptation de la délivrance d'une ou plusieurs fractions ultérieures.

8. Procédé selon la revendication 7, comprenant les étapes consistant à obtenir des informations de dose FLASH délivrée à partir de la mesure à résolution temporelle et des informations de dose FLASH simulée à partir de la simulation à résolution temporelle et l'étape de comparaison comporte la comparaison des informations de dose FLASH délivrée aux informations de dose FLASH simulée.

9. Procédé selon l'une quelconque des revendications 6 à 8, dans lequel l'étape de comparaison comporte la comparaison d'informations de dose cumulée provenant d'un nombre de fractions.

10. Procédé selon l'une quelconque des revendications précédentes, dans lequel l'étape consistant à effectuer une action comporte l'ajustement du modèle du système en fonction du résultat de la comparaison.

11. Procédé selon la revendication 3, dans lequel l'étape consistant à effectuer une action comporte l'évaluation de la fonction du système de délivrance en fonction du résultat de la comparaison.

12. Procédé selon la revendication 1, dans lequel le modèle du système comporte des paramètres en fonction de données nominales fournies pour le système de délivrance ou le modèle du système comporte des paramètres obtenus par une caractérisation expérimentale du système.

13. Produit programme informatique comprenant un moyen de code lisible par ordinateur qui, lorsqu'il est exécuté dans un ordinateur, amènera l'ordinateur à mettre en œuvre le procédé selon l'une quelconque des revendications précédentes.

14. Système informatique comprenant un processeur, une mémoire de programme et facultativement une mémoire de données, dans lequel la mémoire de programme comprend un produit programme informatique selon la revendication 13.
